# EUROPEAN PATENT SPECIFICATION

(11) **EP 0 642 337 B1**
(45) Date of publication and mention of the grant of the patent: **03.09.1997**
(21) Application number: 92910598.9
(22) Date of filing: 29.05.1992
(51) Int. Cl.: A61K 31/135, A61K 31/70, A61K 31/185, A61K 31/425

(54) **DAPSONE AND PROMIN FOR THE TREATMENT OF DEMENTIA**
DAPSONE UND PROMIN ZUR BEHANDLUNG DER DEMENZ
DAPSONE ET PROMINE POUR LE TRAITEMENT DE LA MALADIE D'ALZHEIMER

(43) Date of publication of application: 15.03.1995
(73) Proprietor: THE UNIVERSITY OF BRITISH COLUMBIA, Vancouver, British Columbia, V6T 1Z3 (CA)
(72) Inventor: MCGEER, Patrick, L., Vancouver, British Columbia V6T 1C1 (CA); HARADA, Nobuo, Oku-gun, Okayama 701-45 (JP); KIMURA, Hiroshi, Otsu 520-21 (JP); MCGEER, Edith, G., Vancouver, British Columbia V6T 1C1 (CA); SCHULZER, Michael, Vancouver, British Columbia V6K 1M9 (CA)
(74) Representative: Perani, Aurelio
(86) International application number: CA9200228
(87) International publication number: WO9324118

(56) References cited:
- DE-A- 3 923 088
- DEMENTIA, vol. 3, no. 3, 1992, pages 146-149; P.L. MCGEER ET AL.: 'PREVALENCE OF DEMENTIA AMONGST ELDERLY JAPANESE WITH LEPROSY: APPARENT EFFECT OF CHRONIC DRUG THERAPY'
- 'THE MERCK INDEX', 1989, MERCK & CO., INC., RAHWAY, N.J., US

## Description

This invention pertains to the novel use of 4,4'-diaminodiphenylsulfone and its didextrose sulfonate derivative and closely related anti-lepromatous sulfones in the treatment of dementia (Alzheimer's disease) in human beings.

Alzheimer's Disease is by far the most common cause of primary dementia. Either by itself, or in combination with multiple infarcts, it accounts for almost 80% of all cases. No treatment has been established which will prevent the onset or delay the progression of Alzheimer's Disease. It is possible that known drugs would have an as yet unrecognized efficacy in this respect.

4,4'-diaminodiphenylsulfone (Dapsone™) and its didextrose sulfonate derivative (Promine™) were first shown to have a favourable effect in treating rat leprosy in 1941 (Cowdry and Ruangsiri, Arch. Pathol. 32:632, 1941). Successful clinical trials for the treatment of human leprosy followed and these two components are now the most important anti-leprosy drugs. The two drugs have since been used for treating a variety of skin diseases such as dermatitis herpetiformis and efficacy has been reported in several disorders of presumed autoimmune origin such as rheumatoid arthritis, lupus erythematosus and Behcet's disease. Dapsone™ is a drug that has been used worldwide for over 40 years. It has been found to have few side effects and these are well understood due to extensive experience with patients taking the drug continuously for many years. To the applicants' knowledge, 4,4'-diaminodiphenylsulfone and its didextrose sulfonate derivative, and closely related antilepromatous sulfones have never been used or considered for the treatment of dementia.

Leprosy is no longer a fatal disorder, due in substantial measure to treatment with Dapsone™. It has anti-inflammatory action, which may seem paradoxical for treatment of an infectious disease. However, M. leprae survives in macrophages, and one of the deleterious consequences of the infection, which is reduced by Dapsone, is widespread amyloidosis. Dapsone has also been reported to be effective for the treatment of various presumed autoimmune diseases, including dermatitis herpetiformis, rheumatoid arthritis, temporal arteritis, polymyalgia rheumatica, cutaneous lupus erythematosus, Behcet's disease and polyarteritis nodosa. The alternative antileprosy drugs, clofazimine and rifampicin, have also been reported to have efficacy in anti-inflammatory therapeutic applications.

### SUMMARY OF THE INVENTION

The invention pertains to the use of a substance selected from the group consisting of 4,4'-diaminodiphenylsulfone, glucosulfone sodium (the didextrose sulfonate derivative of 4-4'-diaminodiphenylsulfone), and all closely related sulphone derivatives including, as examples, but not restricted to, sulfoxone sodium, sulfetrone sodium, and thiazolsulfone, and therapeutically and pharmaceutically acceptable salts thereof, in the manufacture of a pharmaceutical formulation for treating dementia in a human being characterized by administering to the human being a therapeutic daily or weekly amount of the formulation.

The substance can be effectively administered to the human being at dosage rates which can vary widely, and on schedules which can vary from twice daily to once weekly. The substance can be either 4,4'-diaminodiphenyl-sulphone, its didextrose sulfonate derivative, other closely related derivatives as mentioned above or pharmaceutically acceptable salts thereof. Typical therapeutic dosages will be in the range of 2 to 20 micromoles per kg body weight per day, and the preferred route will be oral.

The invention also pertains to the use of 4,4'-diaminodiphenyl-sulphone, glucosulfone sodium, and all closely related sulphone derivatives including, as examples, but not restricted to, sulfoxone sodium, sulfetrone sodium, and thiazolsulfone, and therapeutically and pharmaceutically acceptable salts thereof, in the manufacture of a pharmaceutical formulation in a package together with a notification which indicates that the pharmaceutical agent can be used for treating dementia in a human being in a therapeutic amount.

### DRAWINGS

In the drawings:

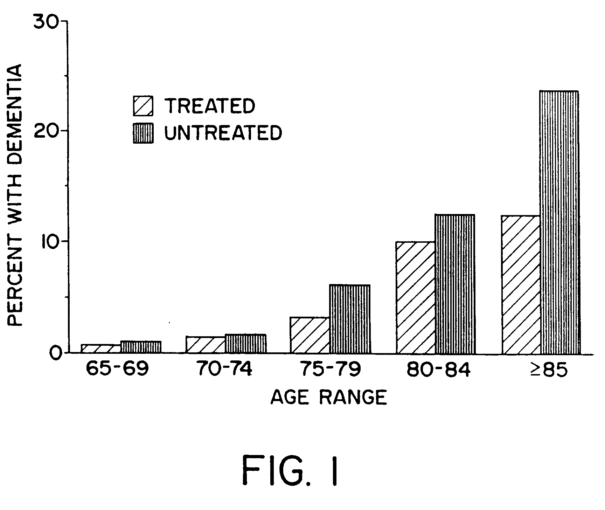

Figure 1 represents a block graphical comparison of dementia percentage among treated and untreated patients from the age of 65 to over 85.

### DETAILED DESCRIPTION OF A PREFERRED EMBODIMENT OF THE INVENTION

Immunohistochemical studies on Alzheimer brain tissue have suggested a chronic inflammatory process may play a role in the observed neuronal degeneration. Dapsone™ 4,4'-diaminodiphenylsulfone, is effective in several chronic inflammatory disorders.

Leprosy patients are almost universally treated with 4,4-diaminodiphenylsulfone (Dapsone™), the didextrose sulfonate derivative (Promin™), or other closely related sulfonic derivatives, over prolonged periods. As a result of such treatment, and the availability of these drugs, leprosy is no longer fatal, and in many cases apparent cures can lead to drug withdrawal. The applicants have noted unexpectedly that the incidence of dementia (Alzheimer disease) amongst elderly leprosy patients being treated with either of the two drugs noted has been unusually low.

The applicants have conducted a formal survey of leprosy hospitals in Japan and have determined that there was significantly less prevalence of dementia amongst Dapsone™ and Promin™ treated leprosy patients compared with those who had been off such drugs for at least five years. It has therefore been concluded that Dapsone™ and Promin™^{,} when administered on a daily dosage basis to the elderly patient, have a preventative action against dementia.

One of the inventors observed that on the Japanese island of Nagashima, leprosy patients seemed to have a low prevalence of dementia. Patients on this island live independently, but are under close medical supervision.

In a survey of thirteen national and three private leprosy hospitals in Japan, the incidence of dementia in patients 65 years and over was determined according to whether they were still receiving continuous treatment with 4,4'-diaminodiphenylsulfone, its didextrose sulfonate derivative or other anti-leprosy drugs, had received intermittent treatment, or no drug treatment with such chemicals over the previous five years.

The overall prevalence of dementia was 2.9% of the 1,410 patients 65 years and over continuously treated cases observed. This compares with 4.83% of 621 intermittently treated cases and 6.25% of 1,761 cases untreated for at least five years. Multiple logistic regression analysis showed a highly significant increase of dementia with age (p = 0.0001) and, after age adjustment, a significant reduction of dementia in patients on continuous drug treatment as compared with patients free of drugs for at least five years (p = 0.017). Treatment had no significant effect on the prevalence of strokes. The dementia figure for untreated cases was virtually double and compared closely with the figure of 6.25% reported by Shibayami et al. for the Japanese population over 65 years of age (Acta. Psychiat. Scand. 74:144-151, 1986). Statistical analysis of these data show that the probability of developing dementia is reduced to 63% by treatment with either Dapsone™ or Promine™. These data show preliminary evidence of the effectiveness of Dapsone™ and Promin™ in retarding the development of dementia.

### Methods

Each institute was asked to categorize their elderly (65 years of age or over) patients according to age range and drug treatment over the past five years (continuously on Dapsone™, Promine™ or other anti-lepromatous drug; free of drug treatment; or on intermittent treatment). In each category, the total number of patients and the numbers with dementia or stroke were to be enumerated. In this survey, no attempt was made to identify the nature of the dementia except that persons who had had a clearly identified stroke in the absence of previous dementia were to be classed in the stroke, rather than the demented groups. In the treatment group overall, 1,240 (88%) had been treated with Dapsone, 53 (3.76%) with Promine, which is the didextrose sulfonate derivative of Dapsone, and the rest with other antileprosy drugs. The percentages of demented were highly similar in these subgroups so that they were combined for analysis. The statistical analysis of the data was complicated by the sharp dependence of dementia on age and the failure of the groups to be precisely age-matched. Accordingly, the data on the treated and untreated groups were subjected to logistic regression analysis to determine their level of significance.

### Results

The results for the treated, untreated and intermittently treated groups are summarized in Table 1:

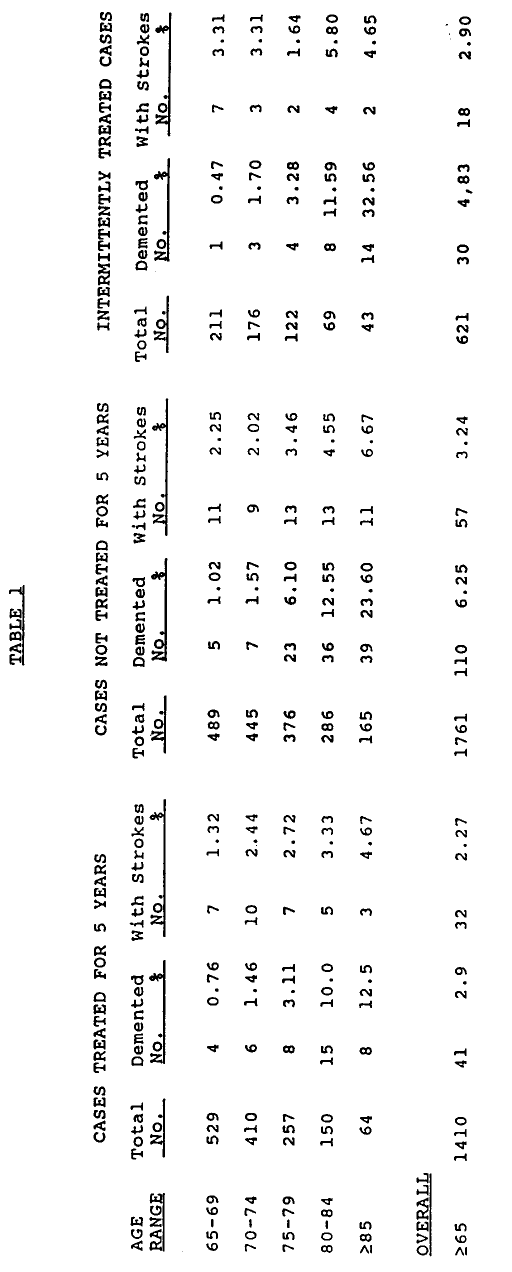

The overall prevalence of dementia was 2.9% of the 1,410 continuously treated cases, 4.83% of the 621 intermittently treated cases, and 6.25% of the 1,761 cases untreated for at least five years. For stroke, the comparative figures were 2.27%, 2.90% and 3.24% respectively.

Statistical analysis showed that:
(a) There is a significant increase of dementia with age (p = 0.0001), with a logistic regression coefficient of 0.1721, but there is no age-by-treatment interaction (p = 0.60). This means that aging has the same effect on the prevalence of dementia in both groups and that the odds of developing dementia in either group increase at an annual rate of 18.8%.
(b) After adjustment for age, the effect of treatment is significant (p = 0.017), with a logistic regression coefficient of -0.4633. This means that the odds of developing dementia at any age are reduced by drug treatment to 63% of the corresponding odds in the untreated group (Figure 1.).
(c) After adjusting for age by logistic regression, treatment had no significant effect on the prevalence of strokes (p = 0.30); age, however, was highly significant (p = 0.0002), with a logistic regression coefficient of 0.0609, meaning that the odds of developing a stroke in either group increase at an annual rate of 6%.

The reason for hypothesizing that anti-inflammatory therapy might be effective in preventing or slowing down the progression of Alzheimer's Disease is the accumulation of evidence suggesting that a chronic inflammatory state of the brain exists in this disease. Reactive microglia, which are rarely seen in normal brain tissue, are abundant in Alzheimer's Disease brain tissue. They strongly express class II major histocompatibility complex (MHC) glycoproteins, Fc receptors, and various β2 integrins which are complement receptors. There are also significant numbers of T cells in the tissue matrix. Brain tissue in affected areas is strongly stained by antibodies to a number of complement proteins, including Clq, C3d, C4d and the membrane attack complex C5b-9. This latter finding suggests that some of the neuronal degeneration in Alzheimer's Disease may be due to bystander lysis.

### Therapeutic Dosage

Dapsone, Promine and closely related sulfones such as sulfoxone sodium, sulfetrone sodium and thiazolsulfone are all preferentially administered in oral tablet or enteric coated capsule form. Parenteral administration is possible, since some derivatives, such as Promine, are highly soluble in water, and can be prepared in ampules as an agueous solution of up to 40% concentration. The dosages of sulfones and methods of administration for the retardation of dementia will be comparable to those now used for the treatment of leprosy and dermatitis herpetiformis. Typical dosages of Dapsone will vary from 100-200 mg once daily, to 200-400 mg twice weekly, to 300-600 mg once weekly. Tablets can be of any convenient size, but typically will be of 100 mg. Promine and other sulfones will be administered in equivalent molecular doses. As in leprosy, the drugs will be continued indefinitely. Lifetime treatment is anticipated.

## Claims

1. The use of 4,4'-diaminodiphenylsulfone, the didextrose sulfonate derivative of 4,4'-diaminodiphenylsulfone (glucosulfone sodium), or other closely related sulfone derivatives such as sulfoxone sodium, sulfetrone sodium and thiazolsulfone, and therapeutically and pharmaceutically acceptable salts thereof, in the manufacture of a pharmaceutical formulation for treating dementia in a human being characterized by administering to the human being a therapeutic daily to weekly amount of the formulation.

2. The use as claimed in claim 1 wherein the formulation is administered to the human being at a dosage rate equivalent to between about 50 mg and 300 mg per day of 4,4'-diaminodiphenylsulfone or its equivalent in molecular concentration for other sulfone derivatives.

3. The use of 4,4'-diaminodiphenylsulfone, the didextrose sulfonate derivative of 4,4'-diaminodiphenylsulfone (glucosulfone sodium) or other closely related sulfone derivatives such as sulfoxone sodium, sulfetrone sodium and thiazolsulfone and therapeutically and pharmaceutically acceptable salts thereof, in the manufacture of a pharmaceutical formulation in a package together with a notification that the pharmaceutical formulation can be used in a therapeutic amount for treating dementia in a human being.

4. The use as claimed in claim 3 wherein the notification indicates that the treatment comprises administering the formulation to the human being at a dosage rate equivalent to between about 50 mg and 300 mg per day of 4,4'-diaminodiphenylsulfone or its equivalent in molecular concentration for other sulfone derivatives.

5. The use as claimed in any one of claims 1 to 4 , wherein the substance is 4,4'-diaminodiphenylsulfone.

6. The use as claimed in any one of claims 1 to 4, wherein the substance is the didextrose sulfonate derivative of 4,4'-diaminodiphenylsulfone.

7. The use as claimed in any one of claims 1 to 4, wherein the substances are other closely related sulfone derivatives including sulfoxone sodium, sulfetrone sodium and thiazosulfone.

## Patentansprüche

1. Verwendung von 4,4'-Diaminodiphenylsulfon, das Didextrosesulfonat-Derivat von 4,4'-Diaminodiphenylsulfon (Glucosulfon-Natrium) oder anderer nahe verwandter Sulfon-Derivate wie Sulfoxon-Natrium, Sulfetron-Natrium und Thiazolsulfon und therapeutisch und pharmazeutisch akzeptabler Salze davon bei der Herstellung einer pharmazeutischen Zubereitung zur Behandlung von Dementia beim Menschen, dadurch gekennzeichnet, daß dem Menschen eine therapeutische täglich oder wöchentlich Menge der Zubereitung verabreicht wird.

2. Verwendung gemäß Anspruch 1, wobei die Zubereitung dem Menschen mit einer Dosis von entsprechend zwischen ungefähr 50 mg und 300 mg pro Tag an 4,4'-Diaminodiphenylsulfon oder dessen Äquivalent an molekularer Konzentration für andere Sulfon-Derivate verabreicht wird.

3. Verwendung von 4,4'-Diaminodiphenylsulfon, das Didextrosesulfonat-Derivat von 4,4'-Diaminodiphenylsulfon (Glucosulfon-Natrium) oder anderer nahe verwandter Sulfon-Derivate wie Sulfoxon-Natrium, Sulfetron-Natrium und Thiazolsulfon und therapeutisch und pharmazeutisch akzeptabler Salze davon bei der Herstellung einer pharmazeutischen Zubereitung in einer Packung zusammen mit einer Mitteilung, daß die pharmazeutische Zubereitung in einer therapeutischen Menge zur Behandlung von Dementia beim Menschen verwendet werden kann.

4. Verwendung gemäß Anspruch 3, wobei die Mitteilung anzeigt, daß die Behandlung die Verabreichung der Zubereitung an den Menschen mit einer Dosis von entsprechend zwischen ungefähr 50 mg und 300 mg pro Tag an 4,4'-Diaminodiphenylsulfon oder dessen Äquivalent an molekularer Konzentration für andere Sulfon-Derivate umfaßt.

5. Verwendung gemäß einem der Ansprüche 1 bis 4, wobei die Substanz 4,4'-Diaminodiphenylsulfon ist.

6. Verwendung gemäß einem der Ansprüche 1 bis 4, wobei die Substanz das Didextrosesulfonat-Derivat von 4,4'-Diaminodiphenylsulfon ist.

7. Verwendung gemäß einem der Ansprüche 1 bis 4, wobei die Substanzen andere nah verwandte Sulfon-Derivate einschließlich Sulfoxon-Natrium, Sulfetron-Natrium und Thiazolsulfon sind.

## Revendications

1. Utilisation de la 4,4'-diaminodiphénylsulfone, du dérivé didextrose sulfonate de la 4,4'-diaminodiphénylsulfone (glucosulfone sodique) ou d'autres dérivés sulfones étroitement apparentés comme la sulfoxone sodique, la sulfétrone sodique et la thiazolsulfone et leurs sels acceptables du point de vue thérapeutique et pharmaceutique, dans la fabrication d'une formulation pharmaceutique pour le traitement de la démence chez un être humain, qui est caractérisée par l'administration à l'être humain d'une quantité thérapeutique quotidienne ou hebdomadaire de la formulation.

2. Utilisation suivant la revendication 1, dans laquelle la formulation est administrée à l'être humain à une dose équivalente à une quantité comprise entre environ 50 mg et 300 mg par jour de 4,4'-diaminodiphénylsulfone ou de son équivalent en concentration moléculaire pour les autres dérivés sulfones.

3. Utilisation de la 4,4'-diaminodiphénylsulfone, du dérivé didextrose sulfonate de la 4,4'-diaminodiphénylsulfone (glucosulfone sodique) ou d'autres dérivés sulfones étroitement apparentés comme la sulfoxone sodique, la sulfétrone sodique et la thiazolsulfone et leurs sels acceptables du point de vue thérapeutique et pharmaceutique, dans la fabrication d'une formulation pharmaceutique dans un conditionnement avec un mode d'emploi qui indique que l'agent pharmaceutique peut être utilisé pour le traitement de la démence chez un être humain en une quantité thérapeutique.

4. Utilisation suivant la revendication 3, dans laquelle le mode d'emploi indique que le traitement comprend l'administration de la formulation à l'être humain à une dose équivalente à une quantité comprise entre environ 50 mg et 300 mg par jour de 4,4'-diaminodiphénylsulfone ou de son équivalent en concentration moléculaire pour les autres dérivés sulfones.

5. Utilisation suivant l'une quelconque des revendications 1 à 4, dans laquelle la substance est la 4,4'-diaminodiphénylsulfone.

6. Utilisation suivant l'une quelconque des revendications 1 à 4, dans laquelle la substance est le dérivé didextrose sulfonate de 4,4'-diaminodiphénylsulfone.

7. Utilisation suivant l'une quelconque des revendications 1 à 4, dans laquelle les substances sont d'autres dérivés sulfones étroitement apparentés incluant la sulfoxone sodique, la sulfétrone sodique et la thiazolsulfone.
